(19) Europäisches Patentamt
European Patent Office
Office européen des brevets

(11) **EP 3 355 237 A1**

(12) **EUROPEAN PATENT APPLICATION**
published in accordance with Art. 153(4) EPC

(43) Date of publication:
**01.08.2018   Bulletin 2018/31**

(51) Int Cl.:
***G06K 9/00*** *(2006.01)*          ***A61B 5/1172*** *(2016.01)*

(21) Application number: **16848794.0**

(22) Date of filing: **04.08.2016**

(86) International application number:
**PCT/KR2016/008615**

(87) International publication number:
**WO 2017/052068 (30.03.2017 Gazette 2017/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority:   **21.09.2015   KR 20150133162**

(71) Applicant: **Hanshin University Industry and Academia Cooperation Foundation Osan-si,Gyeonggi-do 18101 (KR)**

(72) Inventors:
• **YEO, Hyeop Goo**
  **Hwaseong-si**
  **Gyeonggi-do 18440 (KR)**
• **JUNG, Seung Min**
  **Seongnam-si**
  **Gyeonggi-do 13599 (KR)**

(74) Representative: **Michalski Hüttermann & Partner Patentanwälte mbB Speditionstraße 21 40221 Düsseldorf (DE)**

(54) **FINGERPRINT RECOGNITION DEVICE FOR CAPACITANCE SENSING, USING DRIVER HAVING PIPELINE SCAN STRUCTURE**

(57)     A fingerprint recognition device for capacitance detection according to an embodiment of the present invention includes: a fingerprint sensor cell array including a plurality of cells connected to a plurality of row lines and a plurality of column lines, a plurality of column line assemblies constituting a specific number of column lines of the fingerprint recognition cell arrays, a variable cycle clock generator operable to operate a pipeline process performed for each column line belonging to the column line set, and a multi-column line selector for selecting one column line among the column lines of the cell array. The variable cycle clock generator may sequentially control an integration operation for each column line belonging to the column line set at different times.

[fig10]

EP 3 355 237 A1

**Description**

## TECHNICAL FIELD

The present disclosure relates to a fingerprint recognition device for capacitive detection. More particularly, the present disclosure relates to a fingerprint recognition device for capacitive detection using a driver of a pipeline scan structure.

## BACKGROUND ART

Fingerprints have been widely used in many areas of Biometrics due to their high identification rate, security and stability, and currently have substantially individual data. Recently, as the fingerprint recognition technology is automated, a fingerprint recognition system that acquires fingerprints in real time has become necessary.

Recently, as low-cost sensors have appeared, a fingerprint detection sensor used in such a fingerprint recognition system is not limited to a special security device, and is applied to peripherals of a personal computer such as a keyboard and a mouse so that the use range of electronic commerce is gradually expanding.

Therefore, in order to participate in these markets, it is necessary to have easy-to-use, small-scale, low-power, low-cost and high-quality fingerprint detection sensor technology. In the past few decades, many methods have been studied to electrically detect fingerprints, and among the methods announced to date, the main fingerprint detection methods are generally classified into optical type, thermal type, and capacitive type.

Among them, the capacitive type uses the principle that the capacitance varies according to the distance between two electrodes to recognize the ridges and valleys of the fingerprint through the difference between the capacitance generated between the sensing electrode and the fingerprint valley and the capacitance generated between the sensing electrode and the fingerprint ridge, thereby obtaining a fingerprint image. Such a capacitive type may be implemented using standard Complementary Metal Oxide Semiconductor (CMOS) process technology so that the structure is simple, the additional device and the special process are not necessary. Therefore, it has advantages of small size, low power, and low cost. However, since the ridges and valleys of the detected ridges are very small with a few femtofarads and the electrodes formed with indium tin oxide (ITO) in a touch screen panel to which a fingerprint is contacted include parasitic elements such as capacitance and resistance, these parasitic elements may cause severe performance degradation in both touch sensitivity and accuracy.

In addition, the integrator-based fingerprint recognition device for capacitive detection may effectively improve the signal-to-noise ratio (SNR), but require a relatively long time to integrate the signal for a certain period of time.

## DISCLOSURE OF THE INVENTION

## TECHNICAL PROBLEM

The present disclosure provides a fingerprint recognition device for capacitive detection capable of obtaining an image fast while eliminating fixed pattern noise and sufficiently improving SNR by using a driver of a pipeline scan structure.

## TECHNICAL SOLUTION

In accordance with an exemplary embodiment, a fingerprint recognition device for capacitive detection includes: a fingerprint sensor cell array including an n x m matrix of n row lines (n is an integer of 1 or more) and m column lines (m is an integer of 1 or more); m/k column line sets each having k column lines (k is an integer of 1 or more and m or less and m/k is an integer of 1 or more) in the fingerprint recognition cell array as one set; an X, Y address counter configured to generate and output address selection signals for selecting the row line or the column line; and a variable cycle clock generator configured to generate a variable cycle clock signal for performing a reset or evaluation operation at the same time in an ith column (i is an integer of 1 or more and k or less) of each of the column line sets with an arbitrary clock cycle to apply a cock signal to the ith column by receiving an X address selection signal among address selection signals of the X and Y address counter.

The fingerprint recognition device may further include: an X-decoder configured to sequentially incrementing an XDEC signal, which is an output signal, from 1 to m/k (m/k is an integer) by receiving an X address selection signal among the address selection signals of the X and Y address counters and using the received X address selection signal as a clock; and a multi-column line selection unit configured to receive the variable cycle clock signal and an XDEC signal, which is an output signal of the X-decoder, and select one column line among column lines of the cell array.

The X, Y address counter may include a first shift ring counter for sequentially selecting a row line of the fingerprint recognition cell array.

The X, Y address counter may further include a second shift ring counter, wherein the second shift ring counter may output a signal for sequentially incrementing 1 to k (k is an integer of 1 or more and m or less and m/k is an integer of 1 or more), and the variable cycle clock generating unit may generate a variable cycle clock signal for performing a reset or evaluation operation at the same time in a ith column (i is an integer of 1 or more and k or less) of columns of each of the k column line sets with an arbitrary clock cycle by receiving the second shift ring counter output signal.

m/k multi-column line selection units may exist in correspondence to the number m/k of the column line sets and, by the variable cycle clock signal, select only one of cell values by k columns of each of the column line sets.

The multi-column line selection unit may include a second column line selection unit, wherein the second column line selection unit may select only one value among values selected by the m/k first column line selection units in response to the XDEC signal.

In accordance with another exemplary embodiment, a fingerprint recognition method for capacitive detection includes: in a fingerprint sensor cell array including an n x m matrix of n row lines (n is an integer of 1 or more) and m column lines (m is an integer of 1 or more), generating and outputting an address selection signals for selecting the row line or the column line; and in m/k column line sets each having k column lines (k is an integer of 1 or more and m or less and m/k is an integer of 1 or more) in the fingerprint recognition cell array as one set, generating a variable cycle clock signal for performing a reset or evaluation operation at the same time in an ith column (i is an integer of 1 or more and k or less) of each of the column line sets with an arbitrary clock cycle to apply a cock signal to the ith column by receiving an X address selection signal among address selection signals.

The method further includes: sequentially incrementing an XDEC signal, which is an output signal, from 1 to m/k (m/k is an integer) by receiving an X address selection signal among the address selection signals and using the received X address selection signal as a clock; and receiving the variable cycle clock signal and the XDEC signal to select one column line among column lines of the cell array.

## ADVANTAGEOUS EFFECTS

As described above, according to the task of the present invention, the integrator-based fingerprint recognition device for capacitive detection may acquire a fast image by simultaneously evaluating signals for a plurality of cells through a driver of a pipeline scan structure.

In addition, since the fingerprint recognition device for capacitive detection according to the present invention performs a final evaluation by using one analog output, the SNR may be sufficiently improved while eliminating fixed pattern noise, and a fast image may be obtained.

## BRIEF DESCRIPTION OF THE DRAWINGS

FIG. 1 is a block diagram of a fingerprint recognition device according to an embodiment of the present invention.

FIG. 2 is a block diagram of a fingerprint recognition device according to another embodiment of the present invention.

FIG. 3 shows switch control signals applied to fingerprint recognition devices according to an embodiment of the present invention shown in FIG. 1 or FIG. 2.

FIG. 4 is a timing diagram of fingerprint recognition devices according to an embodiment of the present invention.

FIG. 5 is a circuit diagram of a charge transfer circuit for capacitance detection included in fingerprint recognition devices according to an embodiment of the present invention.

FIG. 6 is a circuit diagram of a charge transfer circuit for capacitance detection included in fingerprint recognition devices according to another embodiment of the present invention.

FIG. 7 is a timing diagram showing switch control signals applied to charge transfer circuits for capacitance detection and output voltages according to ridges and valleys of a fingerprint according to an embodiment of the present invention.

FIG. 8 is a schematic diagram of a situation where a charge transfer circuit for capacitive detection shown in FIG. 5 is used in a fingerprint recognition device according to a direct method shown in FIG. 1.

FIG. 9 is a schematic diagram of a situation where a charge transfer circuit for capacitive detection shown in FIG. 6 is used in a fingerprint recognition device according to a direct method shown in FIG. 1.

FIG. 10 is a schematic diagram of a situation where a charge transfer circuit for capacitive detection shown in FIG. 5 is used in a fingerprint recognition device according to a pseudo-direct method shown in FIG. 2.

FIG. 11 is a schematic diagram of a situation where a charge transfer circuit for capacitive detection shown in FIG. 6 is used in a fingerprint recognition device according to a pseudo-direct method shown in FIG. 2.

FIG. 12 is a layout top view of a fingerprint recognition device of the pseudo-direct method shown in FIG. 2.

FIG. 13 is a block diagram of a fingerprint recognition device according to another embodiment of the present invention.

FIG. 14 is a block diagram of a fingerprint recognition device having a sensor array cell of a pipelined structure and a driver structure of the present invention.

FIG. 15 is a view illustrating in more detail a fingerprint recognition device shown in FIG. 14.

FIG. 16 is a view showing a structure of a multi-column line selection unit in more detail.

FIG. 17 is a circuit diagram of a first column line selection unit shown in FIG. 16.

FIG. 18 is a timing diagram of variable cycle clock signals for controlling a sensor array of a pipeline structure according to an embodiment of the present invention.

FIG. 19 is a flowchart of a fingerprint recognition method for capacitive detection according to an embodiment of the present invention.

## MODE FOR CARRYING OUT THE INVENTION

With respect to the embodiments of the present invention disclosed herein, specific structural and functional descriptions are merely illustrated for the purpose of illustrating embodiments of the present invention, and embodiments of the present invention may be embodied in various forms and should not be construed as limited to the embodiments set forth herein.

Various modifications are possible in various embodiments of the present disclosure and specific embodiments are illustrated in drawings and related detailed descriptions are listed. However, this does not limit various embodiments of the inventive concept to a specific embodiment and it should be understood that the inventive concept covers all the modifications, equivalents, and/or replacements of this disclosure provided they come within the scope of the appended claims and their equivalents.

It will be understood that the terms "first" and "second" are used herein to describe various components but these components should not be limited by these terms. The above terms are used only to distinguish one component from another. For example, a first component may be referred to as a second component and vice versa without departing from the scope of the inventive concept.

When it is mentioned that a certain component is "coupled with" or "connected with" another component, it should be understood that the certain component is directly "coupled with" or "connected with" to the other component or a further component may be located therebetween. In contrast, when it is mentioned that a certain component is "directly coupled with" or "directly connected with" another component, it will be understood that a further component is not located therebetween. Other expressions that describe the relationship between components, such as "between" and "directly between" or "adjacent to" and "directly adjacent to", should be interpreted in the same manner.

Terms used in this specification are used to describe specific embodiments, and are not intended to limit the scope of the present invention. The singular expressions include plural expressions unless the context clearly dictates otherwise. Additionally, in various embodiments of the present disclosure, the term "include," "comprise," "including," or "comprising," specifies a property, a region, a fixed number, a step, a process, an element and/or a component but does not exclude other properties, regions, fixed numbers, steps, processes, elements and/or components.

Otherwise indicated herein, all the terms used herein, which include technical or scientific terms, may have the same meaning that is generally understood by a person skilled in the art. In general, the terms defined in the dictionary should be considered to have the same meaning as the contextual meaning of the related art, and, unless clearly defined herein, should not be understood abnormally or as having an excessively formal meaning.

On the other hand, if an embodiment is feasible differently, the functions or operations specified in a particular block may occur differently from the order specified in the flowchart. For example, two consecutive blocks may actually be performed substantially concurrently, and the blocks may be performed backwards depending on the associated function or operation.

Hereinafter, preferred embodiments of the present invention will be described with reference to the accompanying drawings.

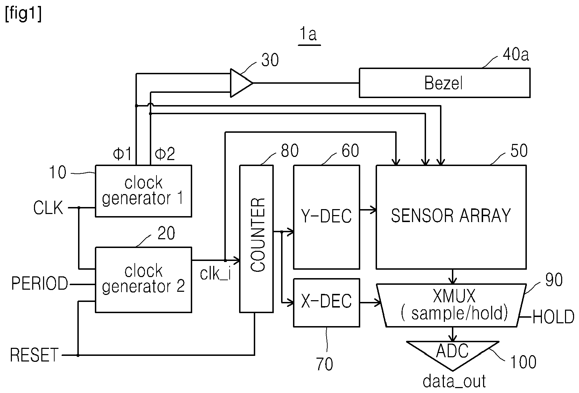

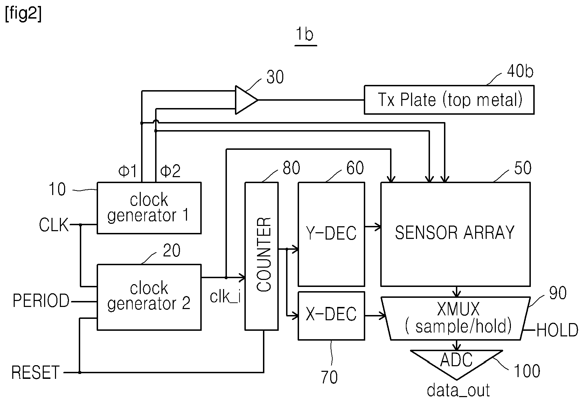

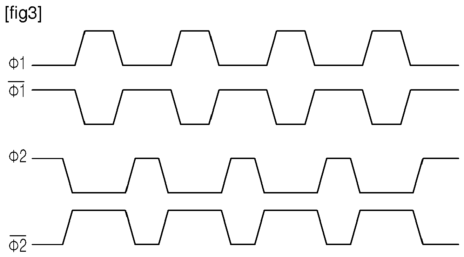

FIG. 1 is a block diagram of a fingerprint recognition device 1a according to an embodiment of the present invention. FIG. 2 is a block diagram of a fingerprint recognition device 1b according to another embodiment of the present invention. FIG. 3 shows switch control signals applied to the fingerprint recognition devices 1a and 1b according to the embodiment of the present invention shown in FIG. 1 or FIG. 2.

Referring to 1, a fingerprint recognition device 1a according to an exemplary embodiment of the present invention includes a first clock generator 10, a second clock generator 20, a bezel driver 30, a bezel 40a, a sensor cell array 50, a Y-drive part 60, an X-drive part 70, a counter, 80, an X-multiplexer 90, and an analog-to-digital converter (ADC) 100.

The first clock generator 10 may generate the first clock signal $\varphi1$ and/or the second clock signal $\varphi2$ in synchronization with the system clock CLK. Also, the first clock generator 10 may generate a first clock bar signal $\overline{\phi1}$ and/or a second clock bar signal $\overline{\phi2}$.

Here, as shown in FIG. 3, the first clock signal φ1 and the second clock signal φ2 may be clock signals that do not overlap with each other. The first clock bar signal $\overline{\phi1}$ is the inverted first clock signal φ1 and the second clock bar signal $\overline{\phi2}$ is the inverted second clock signal φ2.

The second clock generator 20 may generate a signal clk_i, which is a reset/evaluation pulse having a variable cycle, based on a system clock CLK, and generate various cycles according to a signal PERIOD. The second clock generator 20 may start an operation by receiving the signal RESET initially.

The bezel driver 30 may receive the first clock signal φ1 and/or the second clock signal φ2 and output a signal for driving the bezel. In addition, the bezel driver 30 may receive the first clock bar signal $\overline{\phi1}$ and/or the second clock bar signal $\overline{\phi2}$ and output a signal for driving the bezel.

The bezel 40a may be used to apply a power input signal directly to the finger. The bezel 40a may be a metal, an electrically conductive plastic (polymers), or an electrically conductive material. As will be described later, in the present invention, a method of performing an operation by directly touching a finger through a bezel is referred to as a direct method.

The sensor cell array 50 may be a plurality of sensor plates arranged in a matrix of a pixel unit and may have a capacitance formed according to whether the fingerprint portion is in contact with a ridge or a valley.

The Y-drive part 60 may sequentially select and drive sensor cells in each row arranged in a row of the sensor cell array 50. The X-drive part 70 may sequentially select and drive the sensor cells of each column arranged in a column of the sensor cell array 50.

To detect the capacitance of each sensor cell formed in the contact fingerprint, the counter 80 may increase the values of the respective rows and columns of the sensor cell array 50 to sequentially activate each sensor cell.

The X-multiplexer 90 may sample/hold the capacitance value sensed by the sensor cell of one pixel selected in the sensor cell array 50 according to the signal HOLD. The ADC 100 may convert the sampled/held capacitance value from the X-multiplexer 90 into a digital value. Depending on the value of the capacitance converted to a digital value, a microprocessor, a central processor unit (CPU), and an application processor (AP) existing in the outside or inside may determine ridges or valleys.

FIG. 2 is a block diagram of a fingerprint recognition device 1b according to another embodiment of the present invention. The same reference numerals are used for the elements described in the above embodiments of the present invention, and a detailed description thereof will be omitted.

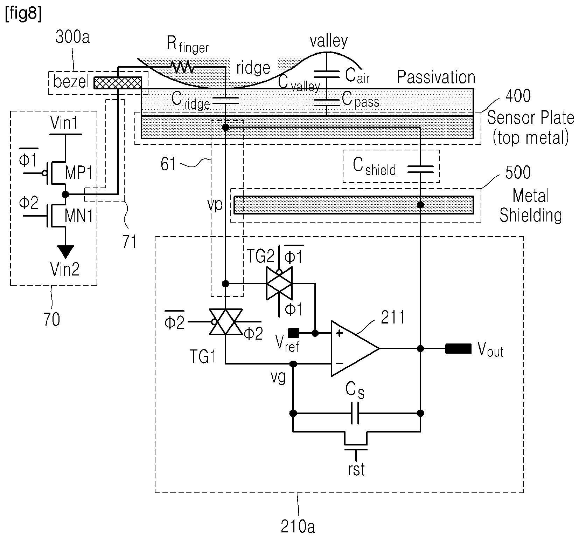

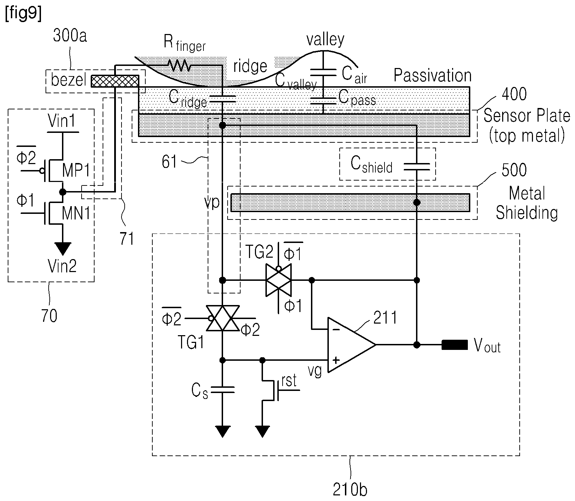

The fingerprint recognition device 1b according to another embodiment of the present invention, which is described with reference to FIG. 2, differs from the fingerprint recognition device 1a described above in that unlike the method using the bezel 40a (300a in FIG. 8) as an electrode, an electrode for applying a power input signal is replaced with a Tx plate 40b (300b in FIG. 9). As described later, in the present invention, a method of performing an operation by touching a finger on the Tx plate 40b through a passivation layer is referred to as a pseudo-direct method.

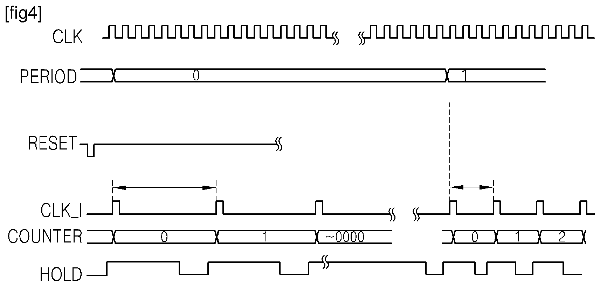

FIG. 4 is a timing diagram of the fingerprint recognition devices 1a and 1b according to the embodiment of the present invention.

Referring to Figs. 1 and 4, when the signal RESET is initially applied to the fingerprint recognition devices 1a and 1b at low, the operation is started. The signal PERIOD is incremented by 1 for each cycle of the counter.

The second clock generator 20 receives the system clock CLK and/or the signal PERIOD and generates a signal CLK_I having a variable cycle. The signal CLK_I may be inputted to generate a reset/evaluation pulse in each sensor cell of the sensor cell array 50. Herein, the reset signal may be a precharge signal. The signal CLK_I may also be inputted to the counter 80.

When the signal CLK_I is high, the selected sensor cell is precharged. When the signal CLK_I is low, the capacitance value of the selected sensor cell may be outputted. The voltage value according to the capacitance value of each sensor cell during one cycle of the signal CLK_I may be sampled and/or held when the signal HOLD is low.

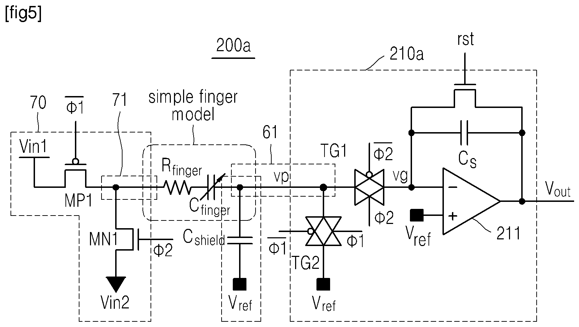

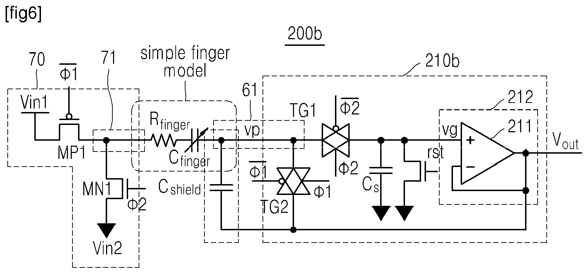

FIG. 5 is a circuit diagram 200a of a charge transfer circuit for capacitance detection included in fingerprint recognition devices 1a and 1b according to an embodiment of the present invention, and FIG. 6 is a circuit diagram 200b of a charge transfer circuit for capacitance detection included in fingerprint recognition devices 1a and 1b according to an embodiment of the present invention.

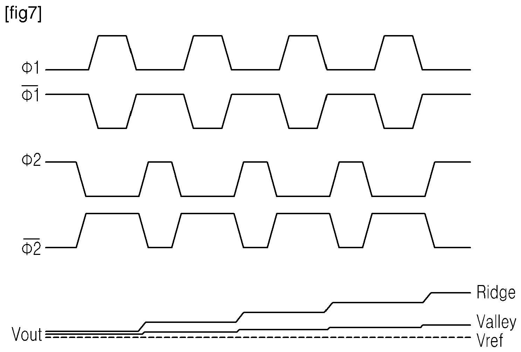

FIG. 7 is a timing diagram showing switch control signals applied to charge transfer circuits 200a and 200b for capacitance detection and an output voltage Vout according to ridges and valleys of the fingerprint according to an embodiment of the present invention.

Referring to FIG 5, the charge transfer circuit 200a for capacitive detection may include an X-drive part 70, an X-drive line 71, a drive line 61, a variable capacitor Cfinger and a parasitic resistance Rfinger defined between the X-drive line 71 and the drive line 61, A parasitic capacitor Cshield generated between a sensor plate and a shielding plate (or metal shielding), and a switched capacitor integrator 210a.

The first switch MP1 and the fourth switch TG2 may be turned on or off by the first clock signal φ1 and the first clock bar signal $\overline{\phi1}$. The second switch MN1 and the third switch TG1 may be turned on or off by the second clock signal φ2 and the second clock bar signal $\overline{\phi2}$.

The parasitic resistance Rfinger and the variable capacitor Cfinger connected in series may be a simple model of the finger being contacted.

The X-drive part 70 may be disposed between one end of the X-drive line 71 opposed to the variable capacitor Cfinger and the voltage input terminal Vin1. The X-drive part 70 may include a first switch MP1 and a second switch MN1.

The first switch MP1 may be disposed between one end of the X-drive line 71 opposed to the variable capacitor Cfinger and the voltage input terminal Vin1. One end of the second switch MN1 may be connected to one end of the X-drive line 71 opposite to the variable capacitor Cfinger, while the other end thereof is connected to the ground.

A predetermined voltage VDD or GND or a reset voltage may be applied to the voltage input terminal Vin1 and/or the ground. Here, the reset voltage may mean that bias may be taken differently for the desired degree of charge transfer. For example, the first power input Vin1 and/or the second power input Vin2 may be inputted to the voltage input terminal Vin1 and/or the ground.

Referring to FIG. 7, the first clock signal φ1 and the second clock signal φ2 together with the first clock bar signal $\overline{\phi 1}$ or the second clock bar signal $\overline{\phi 2}$ may adjust the output voltage Vout of the switches and the switched capacitor integrator 210a.

As shown in FIG. 7, the first clock signal φ1 and the second clock signal φ2 may be clock signals that do not overlap with each other. The first clock bar signal $\overline{\phi 1}$ may be the inverted first clock signal φ1 and the second clock bar signal $\overline{\phi 2}$ may be the inverted second clock signal φ2.

Referring to Figures 5 and 8, as will be described later, a parasitic capacitor Csheild may be generated between shielding plates (or metal shielding) to improve touch sensitivity by blocking the noise generated from the sensor plate connected to the drive line 61 and the circuit under the sensor plate. The circuit under the sensor plate may include a switched capacitor integrator 210a, an active output voltage feedback unit 210b or other circuits. Here, other circuits may include other circuits not related to extracting a variable capacitor Cfinger from a finger.

The switched capacitor integrator 210a may be disposed between the other end of the drive line 61 to which one end of the variable capacitor Cfinger is connected and the voltage output terminal Vout. The switched capacitor integrator 210a may include a third switch TG1 having one end connected to the drive line 61 and the fourth switch TG2 and the other end connected to the inversion input terminal of an operational amplifier 211, a fourth switch TG2 having one end connected to the drive line 61 and the other end connected to the reference voltage input terminal Vref, and the operational amplifier OP-AMP 211 having an inversion input terminal connected to the other end of the third switch TG1 and a non-inversion input terminal to which a reference voltage Vref is inputted.

In addition, it may include a reset switch rst having the other end connected to the voltage output terminal Vout and the other end of the storage capacitor Cs while one end is connected to the other end of the third switch TG1 and the inversion terminal of the operational amplifier 211, and the other end is connected to the storage capacitor Cs connected to the voltage output terminal Vout and one end is connected to the other end of the third switch TG1 and the inversion input terminal of the operation amplifier 211 and one end of the storage capacitor Cs.

Generally, the charges flow from the high voltage side to the low voltage side until the two voltages are equal. Therefore, when charges are transferred from the variable capacitor Cfinger to the storage capacitor Cs, as will be described later, in order to eliminate the parasitic effect of the parasitic capacitor Cshield generated between the sensor plate connected to the drive line 61 and the shielding plate (or metal shielding), it may be required to charge the parasitic capacitor Cshield so that the voltage vp of the drive line 61 is equal to the voltage vg of the inverting input terminal of the operational amplifier 211 (see FIG. 8).

When the first clock signal φ1 is turned on, the output node of the parasitic capacitor Cshield is charged with the reference voltage Vref. The inversion input terminal of the operational amplifier 211 is virtually short-circuited with the non-inversion input terminal to which the reference voltage Vref is inputted.

Therefore, when charges are transferred from the variable capacitor Cfinger to the storage capacitor Cs, since the output node voltage vp of the drive line 61 becomes equal to the inversion input node voltage vg of the operational amplifier 211, no charge is transferred from the parasitic capacitor Cshield to the storage capacitor Cs. As a result, the charges stored in the parasitic capacitor Cshield of the drive line 61 do not affect the output voltage, and the parasitic effect of the parasitic capacitor Cshiled of the drive line 61 may be eliminated.

The transfer function of the charge transfer circuits 200a and 200b for capacitive detection according to an embodiment of the present invention is expressed by Equation 1.

[Equation 1]

$$H(z) = \frac{C_f}{C_s} \cdot \frac{1}{z-1}$$

Here, z is the parameter of the z-transform, Cf is the capacitance of the variable capacitor Cfinger, and Cs is the capacitance of the storage capacitor Cs.

The storage capacitor Cs may integrate the charge transferred from the variable capacitor Cfinger at the rising time of the second clock signal φ2. Here, the slope of the integral value depends on the value of the variable capacitor Cfinger. The value of the variable capacitor Cfinger has a large value in ridge and a small value in valley.

Therefore, as shown in FIG. 7, as the output voltage value Vout of the operational amplifier 211 starts from the reference voltage Vref and the number of clocks increases, in the case of a ridge, it increases more than in the case of a valley.

FIG. 6 is a circuit diagram of a charge transfer circuit 200b for capacitance detection according to another embodiment included in fingerprint recognition devices 1a and 1b according to an embodiment of the present invention. The same reference numerals are used for the elements described in the above embodiments of the present invention, and a detailed description thereof will be omitted.

The charge transfer circuit 200b for capacitive detection according to another embodiment of the present invention described with reference to FIG. 6 is different from the charge transfer circuit 200a for capacitive detection. This will be described below.

The non-inversion input terminal of the operational amplifier 211 constituting the active output voltage feedback unit 210b may be connected to one ends of the third switch TG1, the storage capacitor Cs, and the reset switch rst and the other ends of the storage capacitor Cs and the reset switch rst may be connected to the ground. Then, the other end of the third switch TG1 may be connected to one end of the fourth switch TG2 and one end of the parasitic capacitor Cshield.

The buffer circuit 212 may be a voltage follower that connects the inversion input terminal and the output terminal of the operational amplifier 211.

The inversion input terminal of the operational amplifier 211 may be connected to the output terminal of the operational amplifier 211, the fourth switch TG2, and the other terminal of the parasitic capacitor Cshield. Thus, in order to prevent the charges from being transferred from the parasitic capacitor Cshield, the output voltage Vout may be supplied to the voltage follower and fed back to the fourth switch TG2 and the parasitic capacitor Cshield. When the first clock signal φ1 is in a high state in which the first switch MP1 and the fourth switch TG2 are turned on, the voltage vp of the drive line 61 may be charged to the parasitic capacitor Cshield so as to be equal to the voltage of the buffer circuit input terminal vg. As a result, the charges stored in the parasitic capacitor Cshield do not affect the output voltage. A circuit for performing these operations is referred to as an active output voltage feedback unit 210b in the present invention.

FIG. 8 is a schematic diagram of a situation where the charge transfer circuit for capacitive detection 200a shown in FIG. 5 is used in the fingerprint recognition device 1a according to the direct method shown in FIG. 1.

Referring to FIG. 8, the first clock bar signal $\overline{\phi 1}$ is the inverted first clock signal φ1 and the second clock bar signal $\overline{\phi 2}$ is the inverted second clock signal φ2. The first switch MP1 is a P-channel Metal-Oxide-Semiconductor (MOS) transistor and the second switch MN1 is an N-channel MOS (NMOS) transistor. The third switch TG1 and the fourth switch TG2 are transmission gates in which a PMOS transistor and an NMOS transistor are connected in parallel.

The first switch MP1 and the fourth switch TG2 may be turned on or off by the first clock signal φ1 or the first clock bar signal $\overline{\phi 1}$. The second switch MN1 and the third switch TG1 may be turned on or off by the second clock signal φ2 or the second clock bar signal $\overline{\phi 2}$.

The shielding plate 500 (or metal shielding) prevents the noise generated from the circuit under the sensor plate 400 to improve the touch sensitivity. The circuit under the sensor plate may include the switched capacitor integrator 210a or other circuits independent of extracting a variable capacitor Cfinger from a finger.

At this time, a parasitic capacitor Csheild may be generated between the sensor plate 400 and the shielding plate 500. For example, a parasitic capacitor Cshield may have a range of tens to hundreds of fF depending on the size of the sensor cell. As described with reference to FIG. 5, in order to eliminate the parasitic effect of the parasitic capacitor Cshield generated between the sensor plate 400 connected to the drive line 61 and the shielding plate 500, the switched capacitor integrator 210a may charge the parasitic capacitor Cshield with the voltage vp of the drive line 61 to be equal to the voltage vg of the inversion input terminal of the operational amplifier 211.

When the first clock signal φ1 is high, the output node of the parasitic capacitor Cshield is charged with the reference voltage Vref. The inversion input terminal of the operational amplifier 211 is virtually short-circuited with the non-inversion input terminal to which the reference voltage Vref is inputted.

Therefore, when charges are transferred from the variable capacitor Cfinger to the storage capacitor Cs, since the output node voltage vp of the drive line 61 becomes equal to the inversion input node voltage vg of the operational amplifier 211, the parasitic effect of the parasitic capacitor Cshield may be eliminated.

The bezel 300a may directly apply the power input signal Vin1 or Vin2 in contact with the finger. As described above, in the present invention, a method of performing an operation by directly touching a finger through a bezel is referred to as a direct method.

The bezel 300a may be a metal, an electrically conductive plastic (polymers), or an electrically conductive material.

The finger may be modeled as a parasitic resistance Rfinger and a variable capacitor Cfinger connected in series simply as shown in FIG. 8. Here, the variable capacitor Cfinger may be a ridge capacitor Cridge or a valley capacitor Cvalley.

The sensor plate 400 may be separated from the finger by a passivation layer. Therefore, the finger capacitor Cfinger may be composed of a capacitor in which an air capacitor Cair between the chip surface and the finger skin and a passivation layer capacitor Cpass between the sensor plate 400 and the chip surface are connected in series.

The power input signal Vin1 or Vin2 driven from the driver of the X-drive part 70 is directly drawn to a finger through the bezel contact.

Charges accumulated in the finger capacitors Cfinger may be transferred to the storage capacitors Cs. The finger capacitors Cfinger may have different values depending on ridge capacitors Cridge or valley capacitors Cvalley, respectively.

While the valley capacitor Cvalley corresponds to an air capacitor Cair between the chip surface and the finger skin connected in series and a passivation layer capacitor Cpass between the sensor plate 400 and the chip surface, the ridge capacitor Cridge corresponds to the passivation layer capacitor Cpass between the sensor plate 400 and the chip surface. This may be expressed by the following equation.

[Equation 2]

$$C_{ridge} = C_{pass}$$

[Equation 3]

$$C_{valley} = \frac{C_{air} \cdot C_{pass}}{C_{air} + C_{pass}}$$

The variation of the finger capacitor Cfinger, which is defined as the difference between the ridge capacitor Cridge and the valley capacitor Cvalley, may be expressed as follows.

[Equation 4]

$$\Delta C_{finger} = C_{ridge} - C_{valley}$$

The parasitic capacitor Csheild formed between the sensor plate 400 and the shielding plate 500 may be electrically connected to one end of the fourth switch TG2 and the non-inversion input terminal of the operational amplifier 211. Here, the reference voltage Vref may be inputted to the non-inversion input terminal.

Therefore, when the fourth switch TG2 is turned on, since the voltage of the sensor plate 400 and the voltage of the shielding plate 500 are charged identically with the reference voltage Vref inputted to the non-inversion input terminal of the operational amplifier 211 in the drive line 61, the parasitic capacitor Csheild may be effectively removed. Therefore, the output voltage Vout per single charge transfer of the charge transfer circuit for capacitive detection of the present invention may be expressed as follows.

[Equation 5]

$$V_{out}(t) \cong -\frac{C_{finger}}{C_{finger} + C_S} \cdot V_{dd}$$

Here, the capacitor Cfinger may be a ridge capacitor Cridge or a valley capacitor Cvalley. The transfer function with z as a parameter may be written as following. Here, Vdd may be the input voltage Vin1 or Vin2.

[Equation 6]

$$H(Z) = -\frac{C_{finger}}{C_{finger} + C_S} \cdot \frac{1}{Z - 1}$$

From Equation 5, the output voltages of the ridge capacitors Cridge and the valley capacitors Cvalley may be obtained, and the difference between the output voltages of the ridge capacitors Cridge and the valley capacitors Cvalley may be defined as follows.

[Equation 7]

$$V_{diff} = \left( \frac{C_{ridge}}{C_{ridge} + C_S} - \frac{C_{valley}}{C_{valley} + C_S} \right) \cdot V_{dd}$$

Consequently, the sense voltage Vsense, which is the output voltage difference between the ridge capacitor Cridge and the valley capacitor Cvalley, is defined as follows after the nth integration.

[Equation 8]

$$V_{sense} = n \cdot V_{diff}$$

FIG. 9 is a schematic diagram of a situation where the charge transfer circuit for capacitive detection 200b shown in FIG. 6 is used in the fingerprint recognition device 1a according to the direct method shown in FIG. 1. The same reference numerals are used for the elements described in the above embodiments of the present invention, and a detailed description thereof will be omitted.

The active output voltage feedback unit 210b operates as described with reference to FIG. 6. That is, when the first clock signal φ1 is in a high state in which the first switch MP1 and the fourth switch TG2 are turned on, the parasitic capacitor Cshied may be charged such that the voltage vp of the drive line 61 and the voltage of the buffer circuit input terminal vg of the operational amplifier 211 become the same voltage. Therefore, the parasitic capacitor Cshield may be removed.

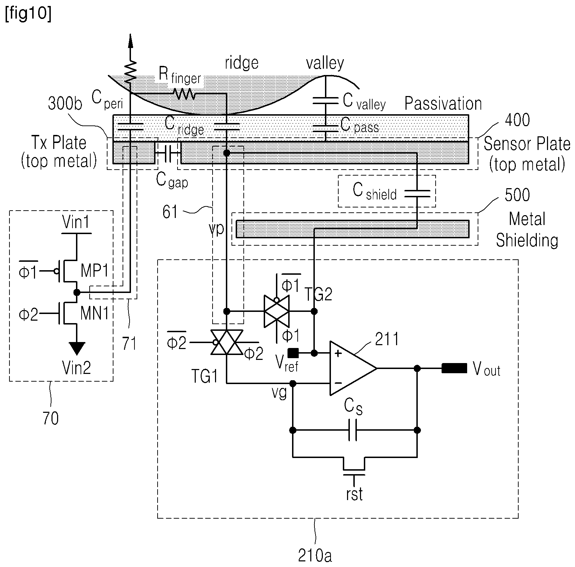

FIG. 10 is a schematic diagram of a situation where the charge transfer circuit for capacitive detection 200a shown in FIG. 5 is used in the fingerprint recognition device 1b according to the pseudo-direct method shown in FIG. 2. The same reference numerals are used for the elements described in the above embodiments of the present invention, and a detailed description thereof will be omitted.

The fingerprint recognition device according to another embodiment of the present invention, which is described with reference to FIG. 10, differs from the fingerprint recognition device (see FIG. 8) described above in that unlike the method using the bezel 300a as an electrode, the electrode for transmitting the power input signal Vin1 or Vin2 is replaced with the Tx plate 300b which is the top metal of the chip. As described later, in the present invention, a method of performing an operation by touching a finger on the Tx plate through a passivation layer is referred to as a pseudo-direct method.

At this time, a passivation layer capacitor Cperi due to a passivation layer may be formed between the Tx plate 300b and the finger. The passivation layer capacitor Cperi may be connected to a ground node and a capacitor Cfinger through a finger and a resistor as shown in FIG. 10.

Therefore, the passivation layer capacitor Cperi and the finger capacitor (Cfinger) may be connected in series, and the total capacitance Ctotal may be expressed by the following equation.

[Equation 9]

$$C_{total} = \frac{C_{peri} \cdot C_{finger}}{C_{peri} + C_{finger}}$$

When it is assumed that the value of the passivation layer capacitor Cperi is larger than that of the finger capacitor Cfinger, it may be seen that the total capacitance Ctotal follows approximately the finger capacitor Cfinger. If the size of the Tx plate 300b is made sufficiently larger than the size of the sensor cell, the influence of the finger capacitor change due to the passivation layer capacitor Cperi may be negligible. In addition, since the pseudo-direct method according to the present invention is based on a switched capacitor integrator or an active output voltage feedback integrator, the voltage output range may be all of the power voltage second.

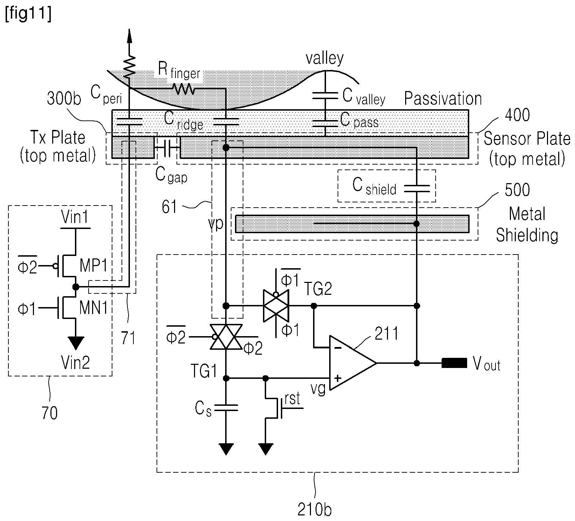

FIG. 11 is a schematic diagram of a situation where the charge transfer circuit for capacitive detection 200b shown in FIG. 6 is used in the fingerprint recognition device 1b according to the pseudo-direct method shown in FIG. 2. The same reference numerals are used for the elements described in the above embodiments of the present invention, and a detailed description thereof will be omitted.

Because it is a pseudo-direct method, unlike the method of using the bezel as an electrode, a top metal Tx plate 300b delivers a power input signal Vin1 or Vin2 to the finger that is in contact through the passivation layer. The operation of the circuit is described with reference to FIG. 6.

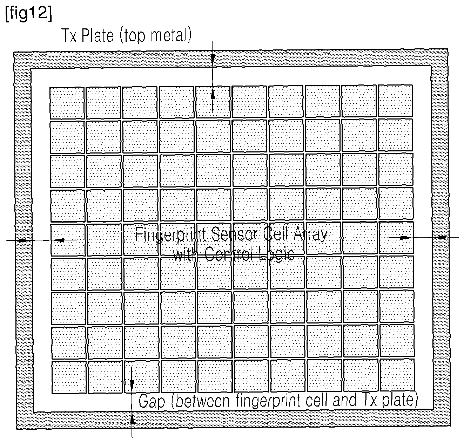

FIG. 12 is a layout top view of a fingerprint recognition device of the pseudo-direct method shown in FIG. 2.

The Tx plate 300b, which is the top metal, surrounds the chip. It is possible to prevent the input signal applied to the Tx plate electrode 300b from being directly transferred to the sensor cell array by making the space capacitor Cgap small enough with a gap from the sensor cell block. This gap may be adjusted appropriately according to the thickness of the passivation layer.

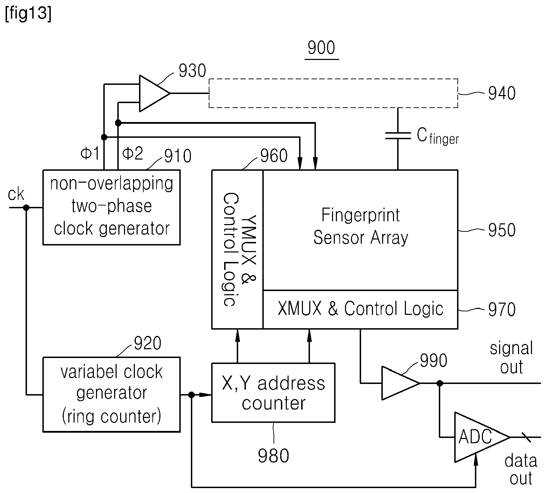

FIG. 13 is a block diagram of a fingerprint recognition device 900 according to another embodiment of the present invention.

Referring to 13, the fingerprint recognition device 900 according to another embodiment of the present invention may include a first clock generator 910, a variable clock generator 920, a bezel driver 930, a bezel 940, a sensor cell array 950, a Y-drive part 960, an X-drive part 970, an X, Y address counter 980, an amplifier 990 and ADC.

The first clock generator 910 may generate the first clock signal φ1 and/or the second clock signal φ2 in synchronization with the system clock CLK. Also, the first clock generator 10 may generate a first clock bar signal $\overline{\phi 1}$ and/or a second clock bar signal $\overline{\phi 2}$. Here, as shown in FIG. 3, the first clock signal φ1 and the second clock signal φ2 may be clock signals that do not overlap with each other. The first clock bar signal $\overline{\phi 1}$ is the inverted first clock signal φ1 and the second clock bar signal $\overline{\phi 2}$ is the inverted second clock signal φ2.

The variable cycle clock generator 920 based on the system clock ck may generate reset/evaluation pulses that amplify the size of the cell signal of the fingerprint sensor array 950 by variably controlling the integration time cycle through the X, Y address counter 980. Meanwhile, the variable cycle clock generator 920 may generate various cycles according to the signal PERIOD as shown in FIG. 1 or FIG. 2, and may start the operation by receiving the signal RESET at the beginning.

The bezel driver 930 may receive the first clock signal φ1 and/or the second clock signal φ2 and output a signal for driving the bezel. In addition, the bezel driver 930 may receive the first clock bar signal $\overline{\phi 1}$ and/or the second clock bar signal $\overline{\phi 2}$ and output a signal for driving the bezel.

The bezel 940 may be used to apply a power input signal directly to the finger. The bezel 940 may be a metal, an electrically conductive plastic (polymers), or an electrically conductive material. Here, as shown in FIGS. 1 and 2, the bezel 940 may be a bezel 40a that performs an operation by directly contacting a finger with a direct method or may be a Tx plate 40b that is the top layer metal of the chip through a passivation layer with the pseudo-direct method.

The sensor cell array 950 may be a plurality of sensor plates arranged in a matrix of a pixel unit and may generate a capacitance Cfinger formed according to whether the touched fingerprint portion is a ridge or a valley.

The Y-drive part 960 may sequentially select and drive sensor cells in each row arranged in a row of the sensor cell array 950.

The X-drive part 970 may drive the capacitance value Cfinger sensed by the sensor cell of one pixel belonging to the row selected by the sensor cell array 950 and the row selected by the Y-drive part 960. Also, to obtain sufficient signal level and SNR, the X-drive part 970 receives the two-phase non-overlapping clock signals φ1 and φ2 shown in FIG. 7 for use in integrating by a significant number of clocks.

To detect the capacitance Cfinger of each sensor cell formed in the contact fingerprint, the X, Y address counter 980 based on the variable cycle clock generated in the variable cycle clock generator 920 sequentially increases the values of each row and each column of the sensor cell array 950 to activate each sensor cell.

An amplifier 990 may amplify the output signal to a larger extend at the signal out terminal. An ADC may be selected from the amplifier 990 and may convert the sampled/held capacitance value to a digital value. Then, depending on the value of the capacitance converted to a digital value, a microprocessor, a central processor unit (CPU), and an application processor (AP) existing in the outside or inside may determine ridges or furrows.

When a signal is transmitted to the finger by the external bezel 940, in relation to the capacitance value stored in each sensor cell, by the switched capacitor integrator 210a shown in FIG. 5 or the active output voltage feedback unit 210b shown in FIG. 6, a sensing signal is accumulated. At this time, in order to obtain satisfactory SNR while obtaining sufficient signal difference between ridges and valleys, as described with reference to FIG. 7, there should be a sufficient integration time, and the SNR according to the integration time may be expressed by the following equation.

[Equation 10]

$$SNR_{n-\mathrm{int}} = 10 \cdot \log\left(\frac{V_s^2}{\sigma^2}\right) = 10\log\left(\frac{(n \cdot \Delta V_{out})^2}{\sigma^2}\right) = SNR_{\sin gle} + 20\log(n)dB$$

Referring to Equation 10, the signal-to-noise ratio increases in proportion to the log scale of the integration time. In order to secure the reliability of the device, the sensing capacitance is decreased in inverse proportion to the thickness when a passivation layer or an additional passivation layer is formed on the chip as shown in FIG. 8. If the passivation layer has a thickness of 100 $\mu$m or more, the capacitance between the ridge and the sensing plate becomes 1 fF or less in a material having a relative dielectric constant of 5 or less.

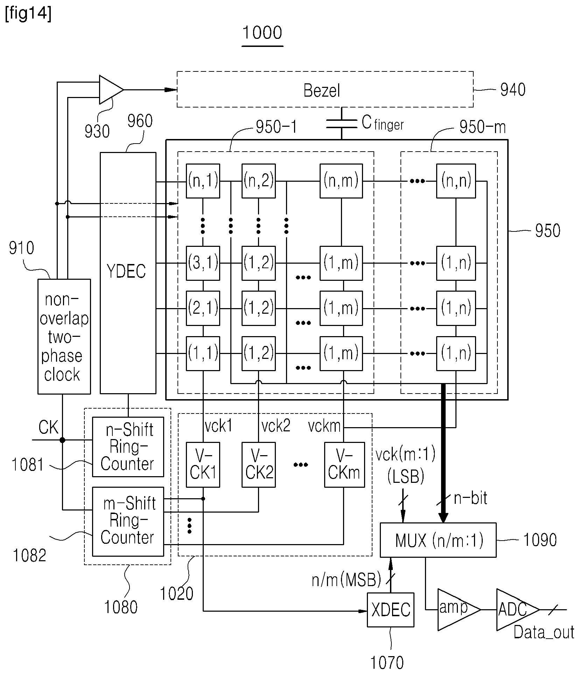

On the other hand, when the variable cycle clock generator 920 increases the SNR by increasing the integration time, it is possible to obtain a stable good quality sensing signal. However, the overall image acquisition time is increased due to an increase in sensing time of each sensor cell. In order to solve such a problem, a sensor array cell of a pipeline structure and a driver structure as shown in FIG. 14 are proposed.

FIG. 14 is a block diagram of a fingerprint recognition device for capacitive detection 1000 having a sensor array cell of the pipelined structure and driver structure of the present invention.

Referring to FIG. 14, a fingerprint recognition device for capacitive detection 1000 includes a first clock generator 910, a variable cycle clock generator 1020, a bezel driver 930, a bezel 940, a sensor cell array 1050, a Y-drive part 960, an X, Y address counter 1080, an X-decoder 1070, an amplifier, and an ADC.

Here, the first clock generator 910, the bezel driver 930, the bezel 940, the sensor cell array 950, the Y-drive part 960, the amplifier, and the ADC 13 are identical to those of FIG. 13 and thus detailed description thereof is omitted. The cell array 950 may be of any size but is conveniently represented by n X n size. The variable cycle clock generation unit 1020 based on the system clock ck constitutes variable cycle clock signals V-CK1 to V-CKm that are counters corresponding to the number m of pipeline processes to control the cells of the X line (or column line). At this time, each of m cells is connected to the same V-CK and controlled. That is, cells of 1, m + 1, 2m + 1, ..., n-m + 1 th X lines are connected to V-CK1. Cells of other X lines have the same structure. That is, it is possible to generate clock signals vck1, vck2,..., Vck m, which are variable cycle clock signals, for controlling reset/evaluation pulses corresponding to the number m of pipeline processes for each column line.

The X, Y address counter 1080 includes an n-shift ring counter 1081 and an m-shift ring counter 1082 to output the output signals of the n-shift ring counter 1081 and the m-shift ring counter 1082 as address selection signals.

The n-shift ring counter 1081 controls the Y-drive part 960 and outputs a Y address selection signal to enable the Y-drive part 960 to sequentially select cells in the Y-line. The m-shift ring counter 1082 outputs an X address selection signal so that the variable cycle clock generator 1020 generates signals vck1, vck2, ..., vckm, which are the variable cycle clock signals, at intervals corresponding to the number of specific clocks.

In such a way, the X, Y address counter 1080 may generate and output address selection signals for selecting a row line or a column line. That is, the X, Y address counter 1080 may sequentially select the cells of a row line Y-line using the output signal of the n-shift ring counter 1081 as a Y address selection signal. In addition, the X, Y address counter 1080 may sequentially select the cells of a column line X-line using the output signal of the m-shift ring counter 1082 as an X address selection signal.

The multi-column line selection unit 1090 receives the variable cycle clock signals as the Least Significant Bit (LSB) and the XDEC output signal of the X-decoder 1070 as the Most Significant Bit (MSB), so that it may finally select one column line and sample/hold the selected capacitance value.

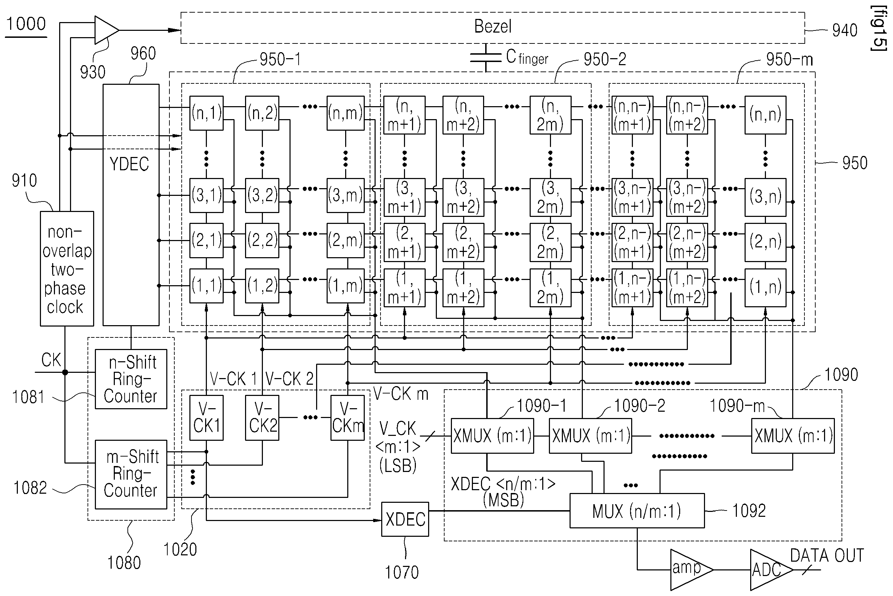

FIG. 15 is a view illustrating in more detail the fingerprint recognition device for capacitive detection 1000 shown in FIG. 14. Referring to FIG. 15, as described above, the variable cycle clock generator 1020 based on the system clock ck constitutes variable cycle clock signals V-CK1 to V-CK m, which are the counters corresponding to the number m of pipeline processes operating at each column line belonging to the sets 950-1, 950-2, ... , 950-m of each column line by the X address selection signal outputted by the m-shift ring counter 1082, to control cells of the X line. At this time, each of m cells is connected to the same V-CK and controlled. That is, cells of 1, m + 1, 2m + 1, ..., n-m + 1 th X lines are connected to V-CK1. Cells of other X lines have the same structure.

That is, it is possible to generate clock signals vck1, vck2,..., Vck m, which are variable cycle clock signals, for controlling reset/evaluation pulses corresponding to the number m of pipeline processes operating at each column line belonging to the sets 950-1, 950-2, ... , 950-m of the column line. By using the variable cycle clock signals of vck1, vck2, ..., vck m, the variable cycle clock generator 1020 may generate reset/evaluation pulses at different times according to the X address selection signal of the m-shift ring counter 1082, and may also arbitrarily control the integration cycle. The X-decoder 1070 provides an XDEC signal as the Most Significant Bit (MSB) signal so that a multi-column line selection unit 1090 may finally select one column line.

By using the X address selection signal output of the m-shift ring counter 1082 that controls the variable cycle clock signals V-CK1, V-CK2, ..., V-CKm of the variable cycle clock generator 1020 as a clock, the X-decoder 1070 counts or sequentially increases an XDEC signal, which is an output signal of the X-decoder 1070, and uses it as to a signal for selecting the second column line selection unit 1092 of the multi-column line selection unit 1090.

The m-shift ring counter 1082 may select a variable cycle clock signal (or signal V-CK) generated by the variable cycle clock generator 1020 at intervals of a fixed number of clocks. For example, the m-shift ring counter 1082 that shifts every eight clocks sequentially selects the first X-th line to the m-th X-th line, and then returns to select the first X-line. At this time, 1, m+1, 2m+1, ..., n-m+lth X lines are selected but the cell of one X-line of the 1, m+1, 2m+1, ..., n-m+lth X lines is selected and a signal is outputted by the multi-column line selection unit 1090.

The multi-column line selection unit 1090 may select specific column lines from the sets 950-1, 950-2, ..., 950-m of a plurality of column lines selected first by the variable cycle clock signals V_CK <m: 1> outputted from the variable cycle clock generator 1020, and may finally select one column line from the specific column lines among the sets 950-1, 950-2, ..., 950-m of a plurality of column lines selected secondarily by receiving XDEC <n/m: 1> which is the output signal of the X-decoder 1070. In addition, the multi-column line selection unit 1090 may sample/hold the capacitance value of the selected cell.

For example, if the third column lines are selected from each column line set among the sets 950-1, 950-2, ..., 950-m of a plurality of column lines selected first, the third column lines are selected from the first column line selection unit 1090-1, the second first column line selection unit 1090-2, the third first column line selection unit 1090-3, ..., mth first column line selection unit 1090-m constituting each column line set. Next, among the firstly selected third column lines, the second column line selection unit 1092 secondarily receives an XDEC signal, which is an output signal of the X-decoder 1070, as an MSB signal and finally, outputs the cell capacitance value of one column line.

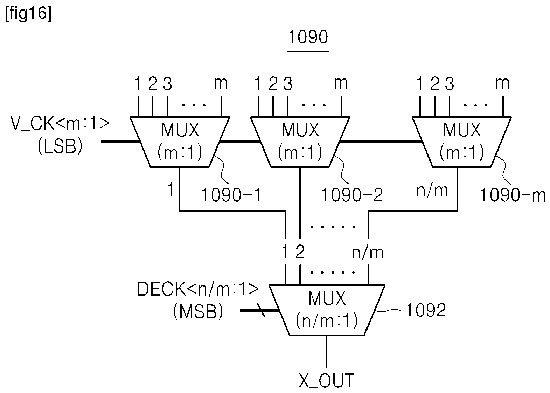

FIG. 16 is a view showing the structure of the multi-column line selection unit 1090 in more detail. Referring to FIG. 16, a multi-column line selection unit 1090 has a two-stage structure. Each of the first column line selection units 1090-1, 1090-2, ..., 1090-m receives m column line inputs and selects one column line input according to each signal V-CK <m:1>, and outputs the selected one to the second column line selection unit 1092.

The second column line selection unit 1092 receives n/m inputs, which are the outputs of the first column line selection units 1090-1, 1090-2, ..., 1090-m, and selects and outputs one. The second column line selection unit 1092 may eventually select one input by receiving n inputs (m X (n/m) = n). The variable cycle clock signal V-CK <m: 1> inputted to each of the first column line selection units 1090-1, 1090-2, ..., 1090-m becomes the LSB selection signal, and the XDEC <n/m: 1> signal, which is the output signal of the X-decoder 1070 and inputted to the second column line selection unit 1092, becomes the MSB selection signal.

Here, the XDEC <n/m: 1> signal, which is the output signal of the X-decoder 1070, is counted or sequentially increased by using the X address selection signal output of the m-shift ring counter 1802 as a clock. In addition, the second column line selection unit 1092 receives the XDEC <n/m: 1> signal, which is the output signal of the X-decoder 1070, and selects one of the output values of the first column line selection units 1090-1, 1090-2, ..., 1090-m.

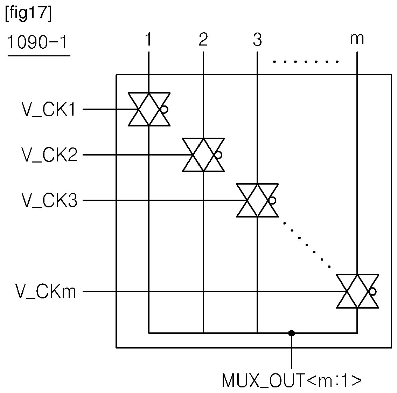

FIG. 17 is a circuit diagram of the first column line selection unit 1090-1 shown in FIG. 16. Referring to FIG. 17, the first column line selection unit 1090-1 includes a CMOS transmission gate and may select one input of the m column line inputs according to the signal V_CK <m: 1>, which is a variable cycle clock signal. The same applies to other first column line selection units 1090-m.

For example, a general capacitive detection fingerprint recognition apparatus 1000 will be described as follows.

If there is a fingerprint sensor cell array for capacitive detection including a matrix of n X m columns composed of n row lines (n is an integer of 1 or more) and m column lines (m is an integer of 1 or more), each first column line selection unit 1090-1, ..., 1090-m has m/k column line sets by using the m/k column lines (k is an integer of 1 or more and m/k is an integer of 1 or more) in the fingerprint recognition cell array for capacitive detection.

The X, Y address counter 1080 may increment n Y addresses sequentially in correspondence to the n low lines by the n-shift ring counter 1081. Also, the X, Y address counter 1080 generates a variable cycle clock signal for performing a reset or evaluation operation at the same time at each ith column (i is an integer of 1 or more and k or less) in correspondence to the k column lines sequentially by the k-shift ring counter (a k-shift ring counter is used since the number of column lines in the column line set is k), and sequentially increments and transmits k X addresses to the variable cycle clock generator 1020 for applying a clock signal to the ith column.

The X-decoder 1070 receives the XDEC signal, which is an output signal sequentially increasing in the k-shift ring counter 1082 of the X, Y address counter 1080, and uses the signal as a clock to increase it from 1 to m/k (m/k is an integer).

Then, the multi-column line selection unit 1090 receives the variable cycle clock signal and the XDEC signal, which is an output signal of the X-decoder, and selects one column line among the column lines of the cell array.

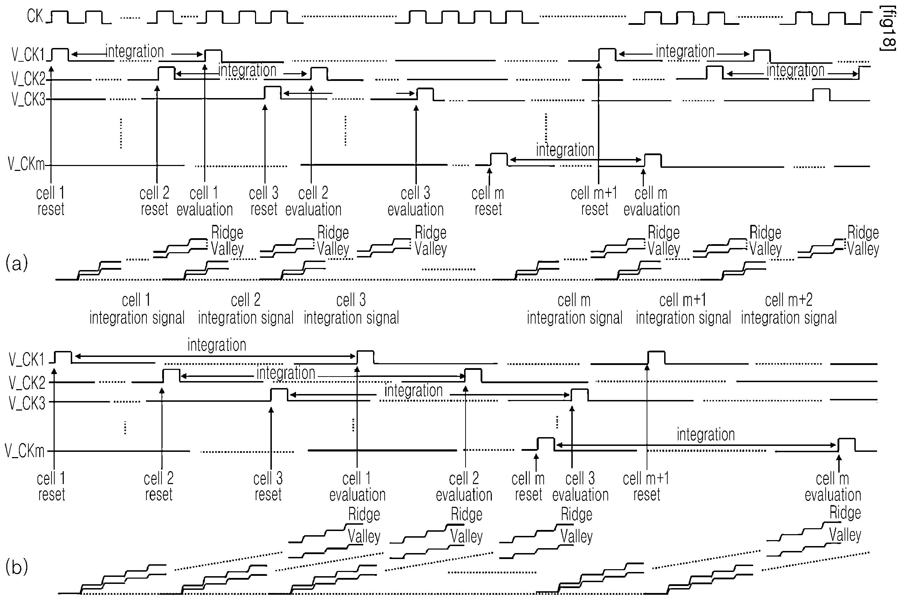

FIG. 18 is a timing diagram of variable cycle clock signals V-CK <m:1> for controlling a sensor array of a pipeline structure according to an embodiment of the present invention. As described above, in order to obtain a signal of sufficient size while increasing the SNR of the sensor cell, the sensing signal should be sufficiently integrated. Referring to FIG. 15, the variable cycle clock generator 1020 may variably change the cycle to control the signal integration cycle of the sensor cell.

Referring to (a) of FIG. 18, V-CK is selected every 8 clocks and a reset/evaluation signal is outputted every 10 clock cycles. V-CK2 operates after 8 clocks after V-CK1 operates, and V-CK operates every 8 clocks after V-CK2 operates. When V-CKm is completed, V-CK1 operates again. Referring to (b) of FIG. 18, in another example of the V-CK clock control, V-CK operates every 8 clocks and outputs a reset/evaluation signal every 20 clock cycles.

In conclusion, proposed is a fingerprint sensor driver structure with a pipeline structure that increases the SNR in proportion to the integration time while effectively improving the problem of the capacitance sensing fingerprint recognition device that requires a long time for fingerprint image acquisition.

Therefore, the integration time may be effectively reduced by integrating the signals of several sensor cells at the same time, and the fixed pattern noise, which occurs when using multiple output analogs, may be effectively eliminated by one analog output. In addition, fast image acquisition may be achieved while improving SNR sufficiently.

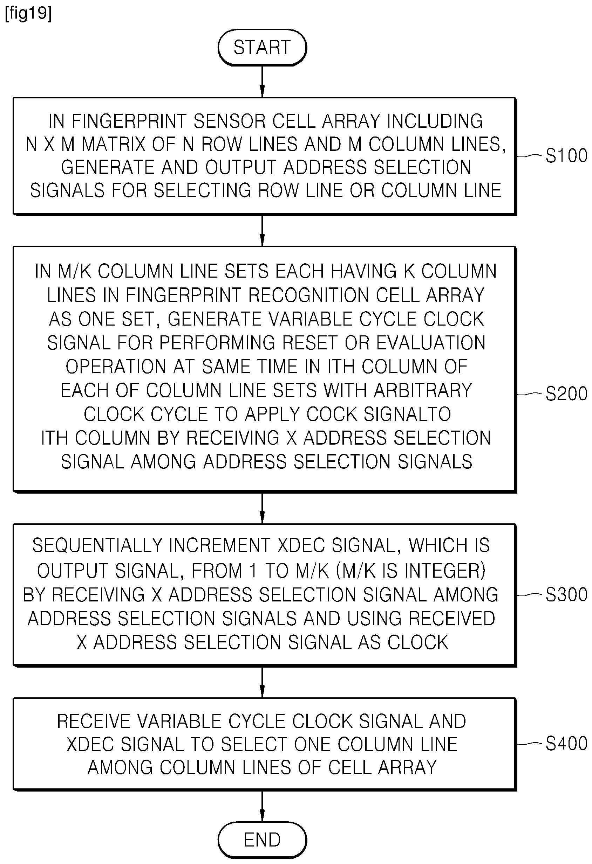

FIG. 19 is a flowchart of a fingerprint recognition method for capacitive detection according to an embodiment of the present invention.

Referring to FIG. 19, in a fingerprint recognition cell array including n x m matrixes of n row lines (n is an integer of 1 or more) and m column lines (m is an integer of 1 or more), the X and Y address counters may generate and output address selection signals for selecting the row line or the column line (S100).

In m/k column line sets (k is an integer of 1 or more and m/k is an integer of 1 or more) using the k column lines as one set by the first column line selection units 1090-1, 1090-2, ..., 1090m, the variable cycle clock generator 1020 receives an X address selection signal among the address selection signals and generates a variable cycle clock signal for performing a reset or evaluation operation simultaneously in each ith column of each of the column line sets, as an arbitrary clock cycle (i is an integer of 1 or more and k or less) to apply the clock signal to the ith column (S200).

Next, the X-decoder 1070 receives the X address selection signal among the address selection signals and uses it as a clock to sequentially increase the XDEC signal, which is an output signal, from 1 to m/k (m/k is an integer) (S300).

Next, the multi-column line selection unit 1090 may receive the variable column clock signal V_CK <m: 1> from the first column line selection units 1090-1 to 1090-m and the XDEC signal from the second column line selection unit 1092, and may select one of the column lines of the cell array (S400).

**Claims**

1. A fingerprint recognition device for capacitive detection comprising:

   a fingerprint sensor cell array including an n x m matrix of n row lines (n is an integer of 1 or more) and m column lines (m is an integer of 1 or more);
   m/k column line sets each having k column lines (k is an integer of 1 or more and m or less and m/k is an integer of 1 or more) in the fingerprint recognition cell array as one set;
   an X, Y address counter configured to generate and output address selection signals for selecting the row line or the column line; and
   a variable cycle clock generator configured to generate a variable cycle clock signal for performing a reset or evaluation operation at the same time in an ith column (i is an integer of 1 or more and k or less) of each of the column line sets with an arbitrary clock cycle to apply a cock signal to the ith column by receiving an X address selection signal among address selection signals of the X and Y address counter.

2. The fingerprint recognition device of claim 1, further comprising:

   an X-decoder configured to sequentially incrementing an XDEC signal, which is an output signal, from 1 to m/k (m/k is an integer) by receiving an X address selection signal among the address selection signals of the X and Y address counters and using the received X address selection signal as a clock; and
   a multi-column line selection unit configured to receive the variable cycle clock signal and an XDEC signal, which is an output signal of the X-decoder, and select one column line among column lines of the cell array.

3. The fingerprint recognition device of claim 2, wherein the X, Y address counter comprises a first shift ring counter for sequentially selecting a row line of the fingerprint recognition cell array.

4. The fingerprint recognition device of claim 3, wherein the X, Y address counter further comprises a second shift ring counter,
   wherein the second shift ring counter outputs a signal for sequentially incrementing 1 to k (k is an integer of 1 or more and m or less and m/k is an integer of 1 or more), and
   the variable cycle clock generating unit generates a variable cycle clock signal for performing a reset or evaluation operation at the same time in a ith column (i is an integer of 1 or more and k or less) of columns of each of the k column line sets with an arbitrary clock cycle by receiving the second shift ring counter output signal.

5. The fingerprint recognition device of claim 4, wherein m/k multi-column line selection units exist in correspondence to the number m/k of the column line sets and, by the variable cycle clock signal, select only one of cell values by k columns of each of the column line sets.

6. The fingerprint recognition device of claim 5, wherein the multi-column line selection unit comprises a second column line selection unit,
   wherein the second column line selection unit selects only one value among values selected by the m/k first column line selection units in response to the XDEC signal.

7. A fingerprint recognition method for capacitive detection, the method comprising:

   in a fingerprint sensor cell array including an n x m matrix of n row lines (n is an integer of 1 or more) and m column lines (m is an integer of 1 or more), generating and outputting an address selection signals for selecting the row line or the column line; and

in m/k column line sets each having k column lines (k is an integer of 1 or more and m or less and m/k is an integer of 1 or more) in the fingerprint recognition cell array as one set, generating a variable cycle clock signal for performing a reset or evaluation operation at the same time in an ith column (i is an integer of 1 or more and k or less) of each of the column line sets with an arbitrary clock cycle to apply a cock signal to the ith column by receiving an X address selection signal among address selection signals.

8.   The method of claim 7, further comprising:

sequentially incrementing an XDEC signal, which is an output signal, from 1 to m/k (m/k is an integer) by receiving an X address selection signal among the address selection signals and using the received X address selection signal as a clock; and
receiving the variable cycle clock signal and the XDEC signal to select one column line among column lines of the cell array.

[fig1]

[fig2]

<u>1b</u>

EP 3 355 237 A1

[fig3]

Φ1
$\overline{\Phi1}$

Φ2

$\overline{\Phi2}$

18

[fig4]

[fig5]

[fig6]

[fig7]

Φ1

$\overline{\Phi1}$

Φ2

$\overline{\Phi2}$

Ridge

Valley

Vout  Vref

[fig8]

[fig9]

[fig10]

[fig11]

[fig12]

Tx Plate (top metal)

Fingerprint Sensor Cell Array
with Control Logic

Gap (between fingerprint cell and Tx plate)

[fig13]

900

930

940

Φ1 Φ2 910

960

C_finger

ck

non-overlapping
two-phase
clock generator

YMUX &
Control Logic

Fingerprint
Sensor Array

950

XMUX & Control Logic

970

920

variabel clock
generator
(ring counter)

X,Y address
counter

990

signal
out

ADC

980

data
out

[fig14]

[fig15]

[fig16]

1090

V_CK<m:1>
(LSB)

1 2 3 ... m    1 2 3 ... m    1 2 3 ... m

MUX (m:1)    MUX (m:1)    MUX (m:1)

1    1090-1    1090-2    n/m    1090-m

1 2 ... n/m

DECK<n/m:1>
(MSB)

MUX
(n/m:1)    1092

X_OUT

[fig17]

1090-1

MUX_OUT<m:1>

(a)

(b)

cell 1
reset

cell 2
reset

cell 1
evaluation

cell 3
reset

cell 2
evaluation

cell 3
evaluation

cell m
reset

cell m+1
reset

cell m
evaluation

cell 1
integration signal

cell 2
integration signal

cell 3
integration signal

cell m
integration signal

cell m+1
integration signal

cell m+2
integration signal

[fig18]

EP 3 355 237 A1

[fig19]

START

↓

IN FINGERPRINT SENSOR CELL ARRAY INCLUDING
N X M MATRIX OF N ROW LINES AND M COLUMN LINES,
GENERATE AND OUTPUT ADDRESS SELECTION
SIGNALS FOR SELECTING ROW LINE OR COLUMN LINE  —S100

↓

IN M/K COLUMN LINE SETS EACH HAVING K COLUMN
LINES IN FINGERPRINT RECOGNITION CELL ARRAY
AS ONE SET, GENERATE VARIABLE CYCLE CLOCK
SIGNAL FOR PERFORMING RESET OR EVALUATION
OPERATION AT SAME TIME IN ITH COLUMN OF
EACH OF COLUMN LINE SETS WITH ARBITRARY
CLOCK CYCLE TO APPLY COCK SIGNALTO
ITH COLUMN BY RECEIVING X ADDRESS SELECTION
SIGNAL AMONG ADDRESS SELECTION SIGNALS  —S200

↓

SEQUENTIALLY INCREMENT XDEC SIGNAL, WHICH IS
OUTPUT SIGNAL, FROM 1 TO M/K (M/K IS INTEGER)
BY RECEIVING X ADDRESS SELECTION SIGNAL AMONG
ADDRESS SELECTION SIGNALS AND USING RECEIVED
X ADDRESS SELECTION SIGNAL AS CLOCK  —S300

↓

RECEIVE VARIABLE CYCLE CLOCK SIGNAL AND
XDEC SIGNAL TO SELECT ONE COLUMN LINE
AMONG COLUMN LINES OF CELL ARRAY  —S400

↓

END

## INTERNATIONAL SEARCH REPORT

International application No.

**PCT/KR2016/008615**

| A. | CLASSIFICATION OF SUBJECT MATTER |
|---|---|

*G06K 9/00(2006.01)i, A61B 5/1172(2016.01)i*

According to International Patent Classification (IPC) or to both national classification and IPC

| B. | FIELDS SEARCHED |
|---|---|

Minimum documentation searched (classification system followed by classification symbols)

G06K 9/00; G06F 3/044; G06K 9/20; G01B 7/28; A61B 5/1172

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: capacitance, fingerprint, address, driver, clock

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 2005-134240 A (ALPS ELECTRIC CO., LTD.) 26 May 2005<br>See paragraphs [0024], [0068] and claim 1. | 1,7 |
| A | | 2-6,8 |
| Y | KR 10-2003-0073508 A (HYNIX SEMICONDUCTOR INC.) 19 September 2003<br>See claims 1-8 and figures 2-3. | 1,7 |
| A | KR 10-1474733 B1 (IMAGIS TECHNOLOGY) 22 December 2014<br>See paragraphs [0030]-[0036], claims 1-3 and figure 2. | 1-8 |
| A | KR 10-1376228 B1 (SILICON DISPLAY TECHNOLOGY) 01 April 2014<br>See paragraphs [0020]-[0033], claims 1-4 and figure 3. | 1-8 |
| A | KR 10-2006-0118087 A (UNIONCOMMUNITY CO., LTD.) 23 November 2006<br>See abstract and claims 1-5. | 1-8 |

☐ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 NOVEMBER 2016 (28.11.2016) | **29 NOVEMBER 2016 (29.11.2016)** |

| Name and mailing address of the ISA/KR<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br><br>Telephone No. |
|---|---|

Form PCT/ISA/210 (second sheet) (January 2015)

# EP 3 355 237 A1

International application No.

**PCT/KR2016/008615**

| Patent document cited in search report | Publication date | Patent family member | Publication date |
|---|---|---|---|
| JP 2005-134240 A | 26/05/2005 | CN 1603846 A | 06/04/2005 |
| | | CN 1603846 C | 04/06/2008 |
| | | EP 1521203 A2 | 06/04/2005 |
| | | EP 1521203 A3 | 27/05/2009 |
| | | JP 2005-114361 A | 28/04/2005 |
| | | JP 4164427 B2 | 15/10/2008 |
| | | US 2005-0073324 A1 | 07/04/2005 |
| | | US 7075316 B2 | 11/07/2006 |
| KR 10-2003-0073508 A | 19/09/2003 | KR 10-0431750 B1 | 17/05/2004 |
| KR 10-1474733 B1 | 22/12/2014 | NONE | |
| KR 10-1376228 B1 | 01/04/2014 | CN 105556538 A | 04/05/2016 |
| | | US 2016-0148036 A1 | 26/05/2016 |
| | | WO 2015-008902 A1 | 22/01/2015 |
| KR 10-2006-0118087 A | 23/11/2006 | KR 10-0672619 B1 | 24/01/2007 |
| | | KR 20-0392690 Y1 | 17/08/2005 |

Form PCT/ISA/210 (patent family annex) (January 2015)